# EUROPEAN PATENT SPECIFICATION

(11) **EP 2 412 344 B1**
(45) Date of publication and mention of the grant of the patent: **05.11.2014**
(21) Application number: 10755780.3
(22) Date of filing: 15.02.2010
(51) Int. Cl.: A61F 13/15, A61F 13/49, A61F 13/494, A61F 13/511, A61F 13/495

(54) **ABSORBENT ARTICLE**
SAUGFÄHIGER ARTIKEL
ARTICLE ABSORBANT

(30) Priority: 25.03.2009 JP 2009075013
(43) Date of publication of application: 01.02.2012
(73) Proprietor: Uni-charm Corporation, Ehime 799-0111 (JP)
(72) Inventor: WAKASUGI, Kei, Kanonji-shi Kagawa 769-1602 (JP); YAGO, Toshiya, Kanonji-shi Kagawa 769-1602 (JP); YAMANAKA, Yasuhiro, Kanonji-shi Kagawa 769-1602 (JP)
(74) Representative: Eke, Philippa Dianne
(86) International application number: PCT/JP2010/052222
(87) International publication number: WO 2010/109979

(56) References cited:
- JP-A- 6 070 957
- JP-A- 2001 061 888
- JP-A- 2001 061 888
- JP-A- 2005 161 006
- US-A- 5 558 660
- US-B2- 6 921 394

## Description

### {Technical Field}

The present invention relates to absorbent articles and more particularly to disposable diapers, toilet-training pants and incontinent briefs.

### {Background}

Conventionally, so-called open-type disposable diapers of which the dorsal region and the ventral region are joined together when being put on the wearer's body are known, for example, from PTL 1 (JP 4049733 B2) . The diaper disclosed therein includes top- and backsheets and an absorbent structure sandwiched between these top- and backsheets. The dorsal region of the diaper is provided along its end with a rear end elastic zone formed of a belt-like elastic member. The dorsal region is further provided along opposite side edges with side elastic zones spaced from each other in a width direction wherein the respective side elastic zones are formed of a plurality of thread-like elastics. The end elastic zone is spaced from the respective side elastic zones in a longitudinal direction to define therebetween a substantially inelastic pocket forming region. Upon contraction of the end elastic zone and the side elastic zones, the pocket forming region is formed with a concave zone adapted to trap baby's loose waste material.

### {Citation List}

### {Patent Literature}

{PTL 1} JP 4049733 B2

### {Summary}

### {Technical Problem}

In the above-cited diaper, the pocket forming region is defined by a large and flexible region extending between the rear end elastic zone and the side elastic zones. Loose waste material being not trapped by the pocket forming region or once trapped but moving toward the dorsal region due to a movement of the wearer such as rolling-over movement comes in direct contact with the rear end elastic zone. The rear end elastic zone creates wrinkles due to its contraction and, in consequence, clearances are formed between the rear end elastic zone and the wearer's back and there is a possibility that loose waste material might leak through such clearance.

An object of this invention is to provide an absorbent article improved to prevent loose waste material or the like from leaking out of the article.

### {Solution to Problem}

The present invention provides the absorbent article of independent claim 1. The dependent claims specify preferred but optional features. According to this invention, there is provided an absorbent article having a longitudinal direction and a transverse direction and including: a chassis including a body-facing side, a garment-facing side, a front waist region, a rear waist region and a crotch region extending between the front and rear waist regions, a liquid-absorbent structure lying at least in the crotch region, rear waist elastics extending in the transverse direction along either the rear end or the front and rear ends of the chassis and leg elastics extending in the longitudinal direction along opposite side edges of the chassis extending in the longitudinal direction; and a pair of containment cuffs lying on the body-facing side of the chassis and extending in the longitudinal direction along the chassis' opposite side edges wherein the containment cuffs include cuffs' front and rear ends bonded to the chassis by means of front and rear ends' bond zones, cuffs' outer edges bonded to the chassis by means of opposite side bond zones and cuffs' inner side edges elasticized so as to be contractible in the longitudinal direction and adapted to be spaced from the chassis.

This invention is **characterized in that** the chassis further includes an inner sheet lying on the body-facing side and an outer sheet lying on the garment-facing side wherein the inner sheet is contoured by front and rear ends extending in the transverse direction and opposite side edges extending in the longitudinal direction and the containment cuffs are located on the body-facing side of the inner sheet; and the liquid-absorbent structure includes front and rear ends extending in the transverse direction, a spacing zone is defined between the inner sheet's rear end and the liquid-absorbent structure's rear end in the longitudinal direction, and the cuffs' inner side edges are bonded to the inner sheet by means of inner bond zones formed in the spacing zone.

The inner sheet has a plurality of ridges extending in the longitudinal direction and grooves each formed between each pair of the ridges being adjacent in the transverse direction.

According to one embodiment of this invention, the containment cuffs are elasticized in the longitudinal direction by cuff elastics attached to the cuffs' inner side edges and the cuff elastics are attached to the cuffs' inner side edges inside the inner bond zones as viewed in the longitudinal direction.

According to still another embodiment of this invention, the paired inner bond zones have the same length dimension as measured from the inner sheet's rear end in the longitudinal direction.

According to yet another embodiment of this invention, the inner sheet has a transverse stiffness against bending in the direction orthogonal to the ridges and the grooves higher than a longitudinal stiffness against bending along the ridges and the grooves wherein the transverse stiffness is more than or equal to 50mm and less than 90mm. The respective stiffness values were measured by the method specified by JIS L1018 8. 22. 1 A.

According to further another embodiment of this invention, the rear waist elastics are attached between the inner sheet and the outer sheet.

### {Advantageous Effects of Invention}

The chassis of the absorbent article is formed of the inner and outer sheets and is formed on the body-facing side of the inner sheet with the containment cuffs. The spacing zone is formed between the inner sheet's rear end and the liquid-absorbent structure's rear end and the cuffs' inner side edges elasticized in the longitudinal direction and the inner sheet are bonded together by means of the inner bond zones which are formed in the spacing zone. With such an arrangement, the inner sheet is pulled inward in the longitudinal direction upon contraction of the cuffs' inner side edges and folded about the inner ends of the respective inner bond zones to form a fold. The fold is formed inside the rear waist elastics as viewed in the longitudinal direction and functions to restrict movement of body waste in the longitudinal direction. In this way, it is ensured that body waste is prevented from leaking out from the absorbent article through clearances formed due to contraction of the rear waist elastics.

### {Brief Description of Drawings}

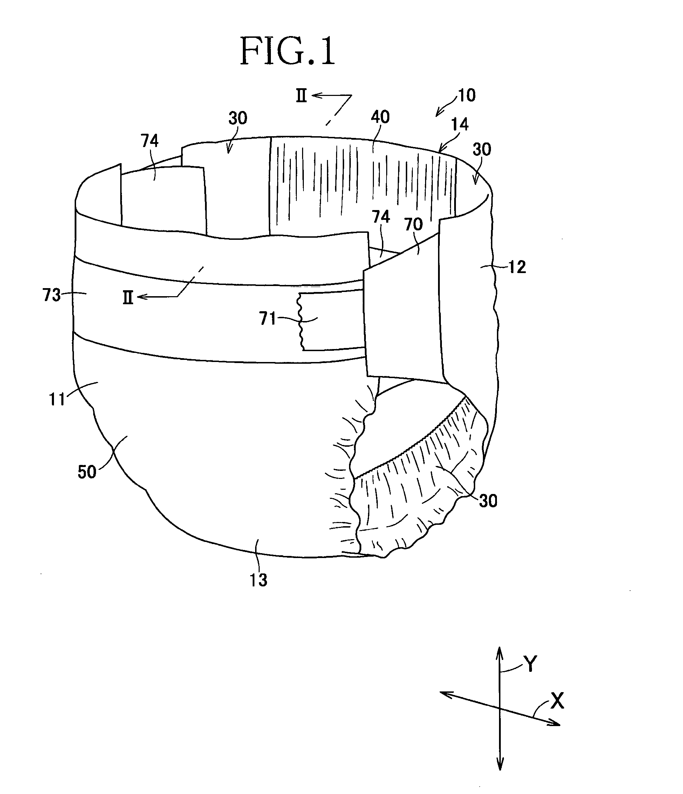

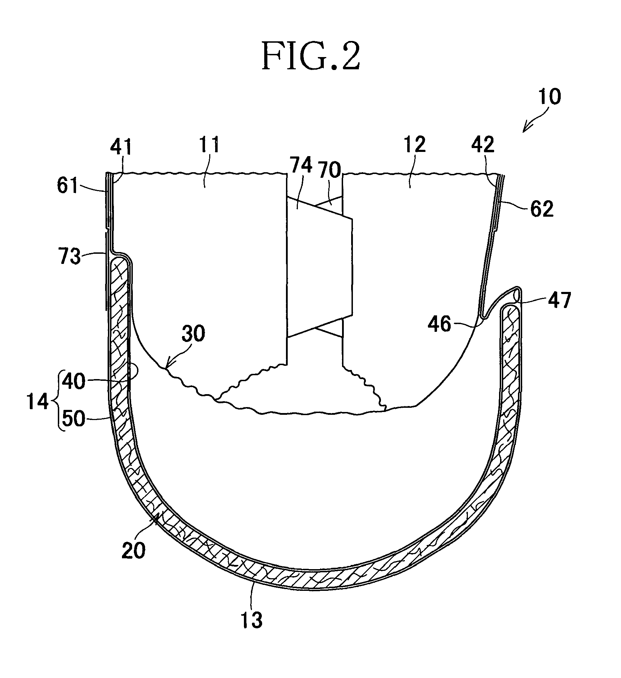

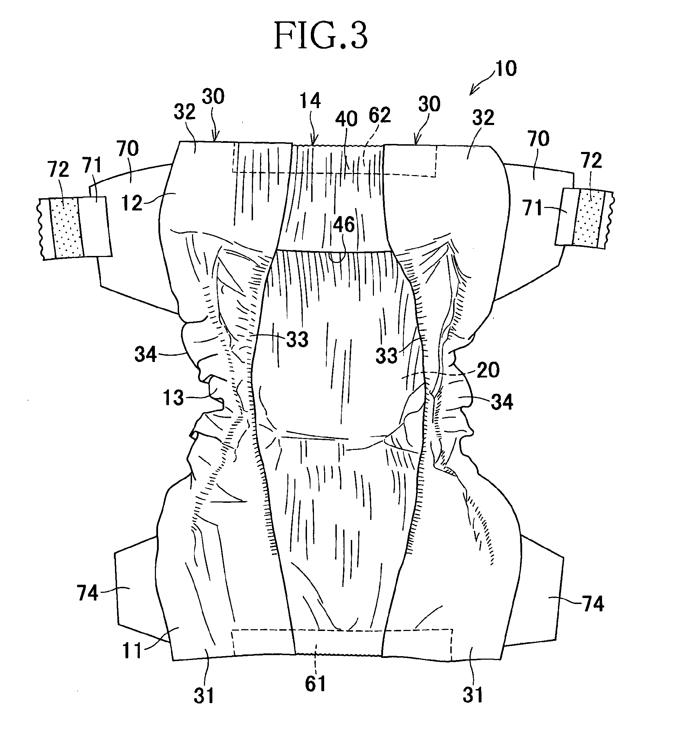

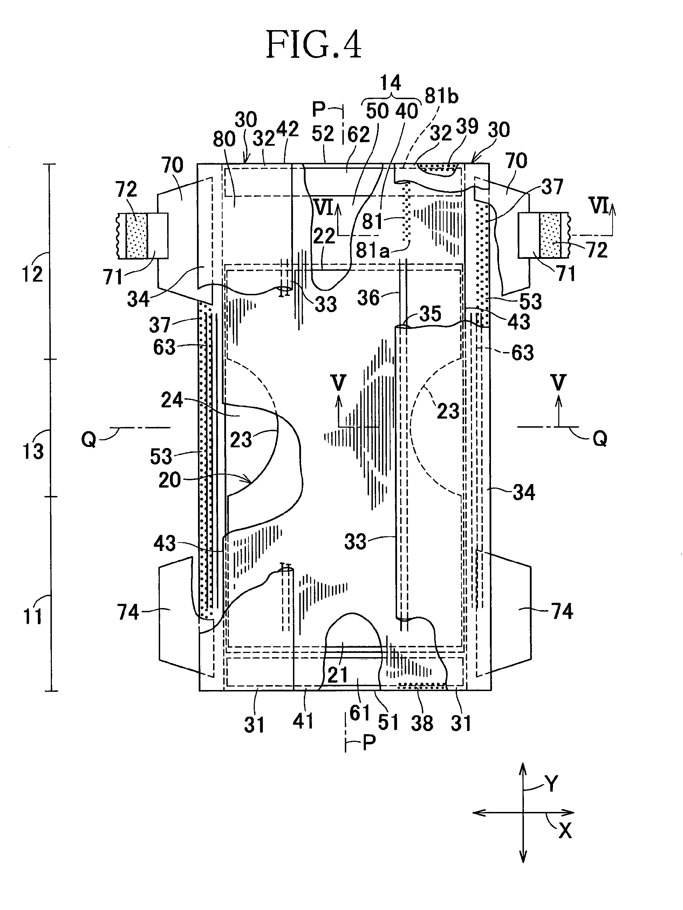

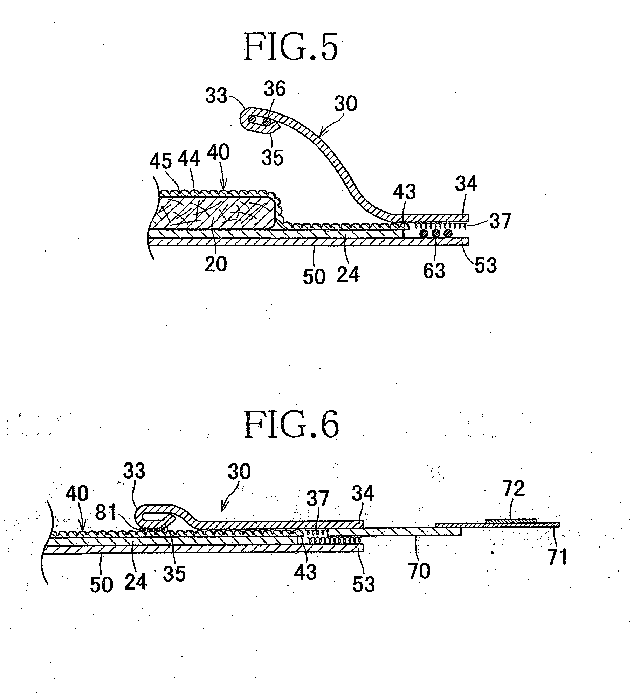

{Fig. 1} A perspective view of a disposable diaper as an example of the absorbent article.
{Fig. 2} A sectional view taken along line II-II in Fig. 1.
{Fig. 3} An open view of the diaper shown in Fig. 1.
{Fig. 4} A plan view corresponding to Fig. 3.
{Fig. 5} A sectional view taken along line V-V in Fig. 4.
{Fig. 6} A sectional view taken along line VI-VI in Fig. 4.

### {Description of Embodiments}

Taking a disposable diaper as an example of the absorbent article, this invention will be described hereunder.

Figs. 1 through 6 show one embodiment of this invention wherein Fig. 1 is a perspective view showing the diaper 10 in its assembled state, Fig. 2 is a sectional view taken along line II-II in Fig. 1, Fig. 3 shows the diaper 10 having front and rear waist regions thereof disengaged from each other, Fig. 4 is aplan view of the diaper 10 having respective elastics stretched against contractile force thereof from the state of Fig. 3 and thereby kept flattened, Fig. 5 is a sectional view taken along line V-V in Fig. 4 and Fig. 6 is a sectional view taken along line VI-VI in Fig. 4. The plan view in Fig. 4 is partially cutaway for convenience of illustration.

The diaper 10 has a longitudinal direction Y and a transverse direction X and is composed of a chassis 14 including a body-facing side, a garment-facing side, a front waist region 11, a rear waist region 12 and a crotch region 13 extending between the front and rear waist regions 11, 12 so as to be contiguous to one another in the longitudinal direction Y; a liquid-absorbent structure extending across the crotch region 13 into the front and rear waist regions 11, 12; and a pair of containment cuffs 30 lying on the body-facing side of the chassis 14. The diaper 10 has an imaginary longitudinal center line P-P bisecting a length dimension in the transverse direction X and an imaginary transverse center line Q-Q bisecting a length dimension in the longitudinal direction Y wherein the diaper 10 has its shape substantially symmetric about the imaginary longitudinal center line P-P.

The chassis 14 includes an inner sheet 40 lying on the body-facing side and an outer sheet 50 lying on the opposite side. Referring to Fig. 4, the inner and outer sheets 40, 50 respectively include front and rear ends 41, 42, 51, 52 and inner side edges 43, 53 wherein a length dimension of the inner sheet 40 in the transverse direction X is smaller than that of the outer sheet 50 so that the outer side edges 53 extend outward beyond the inner side edges 43 and the outer side edges 53 define opposite side edges of the chassis 14. The inner front and rear ends 41, 42 are substantially aligned with the outer front and rear ends 51, 52, respectively, so as to define front and rear ends of the chassis 14.

In the rear waist region 12, the outer sheet 50 includes a pair of rear side sheets 70 attached thereto so as to extend outward in the transverse direction X beyond the opposite side edges 53, respectively, and the respective rear side sheets 70 are formed with hook elements 72 by the intermediary of tab members 71. In the front waist region 11, the outer sheet 50 includes a pair of front side sheets 74 attached thereto so as to extend outward in the transverse direction X beyond the opposite side edges 53, respectively, and these front side sheets 74 are provided in zones defined inside the opposite side edges 53 as viewed in the transverse direction X and on the garment-facing side thereof with loop elements 73 extending in the transverse direction X (See Fig. 1) adapted to be engaged with the associated hook elements 72. The hook elements 72 may be engaged with the associated loop elements 73 to form the pants-type diaper 10.

Referring to Figs. 5 and 6, the inner sheet 40 has a plurality of ridges 44 extending in the longitudinal direction Y and a plurality of grooves 45 each formed between each pair of the adjacent ridges. A thickness dimension of the ridges 44 is in a range of about 1.0 to 3.0mm and about 1.3mm in this embodiment. A thickness dimension of the grooves is in a range of about 0.4 to 1.5mm and about 0.75mm in this embodiment. These thickness dimensions are based on the measurement result obtained by Keyence's Laser Displacement Gauge (KEYENCE LK-G30). Specifically, a distance from a laser irradiation port to a table on which the inner sheet 40 is placed was set to 30.5cm and a length range of 26mm along the ridges 44 or the grooves 45 was measured. The thickness dimension of the ridge 44 corresponds to a length dimension from the table to a crest apex defined by the ridge 44 and to an average of values obtained by measurement conducted over the length of 26mm. The thickness dimension of the groove 45 corresponds to a length dimension from the table to the bottom thereof and to an average of values obtained by measurement conducted over the length of 26mm. A pitch defined between the adjacent ridges 44 was about 4mm.

Such inner sheet 40 is easily bent along the grooves 45, i.e., in the longitudinal direction but not so in a direction which is orthogonal to the grooves 45, i.e., in the transverse direction. A stiffness exhibited by the inner sheet 40 when it is bent along the ridges 44 and the grooves 45 is referred to as a longitudinal stiffness and a stiffness exhibited by the inner sheet 40 in a direction orthogonal thereto is referred to as a transverse stiffness. The longitudinal stiffness is more than or equal to 100mm and less than 140mm, and is about 120mm in this embodiment. The transverse stiffness is more than or equal to 50mm and less than 90mm, and is about 70mm in this embodiment. The longitudinal stiffness is preferably set to be about 1.7 times higher than the transverse stiffness. The respective stiffness values were measured by the method specified by JIS L1018 8. 22. 1 A.

The chassis 14 as has been described above is provided along the front and rear ends with front and rear waist elastics 61, 62 attached thereto under tension and in a contractible manner. The front and rear waist elastics 61, 62 are preferably provided in the form of belt-like elements each made of foamed polyurethane containing open cells and bonded to at least one of the inner and outer sheets 40, 50 by bonding means such as adhesives (not shown). The longitudinal stiffness along the ridges 44 and the grooves 45 of the inner sheet 40 is sufficiently low to be easily contracted in the transverse direction X and the inner sheet 40 should not noticeably interfere with contraction of the front and rear waist elastics 61, 62.

The chassis 14 is provided along its opposite side edges with leg elastics 63 extending in the longitudinal direction Y attached thereto under tension and in a contractible manner. Each of the leg elastics 63 is formed of a plurality of elastic yarns or threads sandwiched between the outer sheet 50 and the containment cuff 30 and bonded to at least one of them by bonding means such as adhesives. The leg elastics 63 are secured to the outer sheet 50 and/or the containment cuffs 30 outside the inner sheet 40 as viewed in the transverse direction X and therefore the inner sheet 40 having a relatively high transverse stiffness in the direction orthogonal to the ridges 44 and the grooves 45 should not interfere with contraction of the leg elastics 63.

A liquid-absorbent structure 20 is disposed between the inner and outer sheets 40, 50. The liquid-absorbent structure 20 is contoured by front and rear ends 21, 22 extending in the transverse direction X and opposite side edges 23 extending in the longitudinal direction Y wherein the front end 21 lies in the front waist region 11 so as to be substantially adjacent to the front waist elastics 61 in the longitudinal direction Y. The rear end 22 of the liquid-absorbent structure lies in the rear waist region 12 and is attached thereto so as to be spaced from the rear waist elastics 62 in the longitudinal direction Y to define a spacing zone 80. The spacing zone 80 is defined between the rear waist elastics 62 and the rear end 22 of the liquid-absorbent structure, in which the liquid-absorbent structure 20 is not present. A length dimension of the spacing zone 80 in the longitudinal direction Y is about 36mm.

A distance between the opposite side edges 23 in the transverse direction X is gradually reduced toward the imaginary longitudinal center line P-P in the crotch region 13 so as to define concave segments. The liquid-absorbent structure 20 includes a liquid-absorbent core made of, for example, a mixture of fluff wood pulp and superabsorbent polymer particles and a liquid-dispersant sheet such as tissue paper used to wrap the liquid-absorbent core. Between the liquid-absorbent structure 20 and the outer sheet 50, a liquid-impervious leakage-barrier sheet 24 formed, for example, of a film is sandwiched.

The containment cuffs 30 provided closer to the wearer's body than the inner sheet 40 respectively include cuffs' front and rear ends 31, 32 and cuffs' inner and outer side edges 33, 34 wherein the cuffs' outer side edges 34 extend outward in the transverse direction X beyond the associated inner side edges 43 and bonded to the associated outer side edges 53 by means of associated side bond zones 37. The leg elastics 63 are secured between the cuffs' outer side edges 34 and the side edges 53 of the outer sheet also by means of the associated side bond zones 37. The cuffs' front and rear ends 31, 32 are substantially aligned with the inner front and rear ends 41, 42 and bonded to the inner front and rear ends 41, 42 by means of front and rear bond zones 38, 39, respectively. The inner side edges 33 of the respective cuffs are partially folded back toward the inner sheet 40 so as to form sleeves 35 within which cuff elastics 36 extending in the longitudinal direction Y are attached in a contractible manner. The respective cuff elastics 36 are formed of a plurality of elastic yarns or threads and attached to the respective cuffs under tension and in a contractible manner wherein the respective cuff elastics 36 are spaced by a predetermined distance from the cuffs' front and rear ends 31, 32 so that the cuffs' inner side edges 33 may be elasticized in the longitudinal direction Y at least in the crotch region 13. It is possible to attach such as an elastic nonwoven fabric to the cuffs' inner side edges 33 and thereby to elasticize the containment cuffs 30 in the longitudinal direction Y.

Between the sleeves 35 formed along the cuffs' inner side edges 33 and the inner sheet 40, is a pair of inner bond zones 81 in which the sleeves 35 are bonded to the inner sheet 40. The inner bond zones 81 extend from the rear end 42 of the inner sheet toward the imaginary transverse center line Q-Q to the spacing zone 80. In other words, inner ends 81a of the respective inner bond zones 81 are defined in the spacing zone 80 and outer ends 81b thereof are defined at positions corresponding to the rear end 42 of the inner sheet. Specifically, the inner ends 81a are positioned at a distance of about 20mm from the rear waist elastics 62.

According to this embodiment, the inner bond zones 81 and the cuff elastics 36 are relatively positioned in such a manner that the bond zones 81 and the cuff elastics 36 do not overlap directly or indirectly and the inner ends 81a are spaced from the cuff elastics 36. These paired inner bond zones 81 have substantially the same length dimension in the longitudinal direction Y thereof and positions of the inner ends 81a are aligned with each other in the transverse direction X.

In the diaper described above, the cuffs' inner side edges 33 are pulled inward in the longitudinal direction Y toward the imaginary transverse center line Q-Q upon contraction of the cuff elastics 36. The cuffs' inner side edges 33 are bonded to the inner sheet 40 in the inner bond zones 81 and therefore, in these bond zones 81, the inner sheet 40 also is pulled toward the imaginary transverse center line Q-Q. The inner sheet 40 is adapted to have relatively high transverse stiffness in the direction orthogonal to the ridges 44 and grooves 45 and consequentially the inner sheet 40 is folded upward toward the wearer's body about the inner ends 81a of the respective inner bond zones 81 to form a fold 46 (See Figs. 2 and 3) as the inner sheet 40 is pulled together with the cuffs' inner side edges 33. Formation of the fold 46 makes a portion of the inner sheet 40 closer to its rear end 42 than the fold 46 to form a folded trough 47 on the garment-facing side. In this way, this fold 46 and trough 47 cooperate with each other to define a pocket.

Even when body waste such as loose waste material moves to the wearer's dorsal side, the fold 46 formed in the above-mentioned manner functions as a barrier to prevent the loose waste material from further moving toward the inner rear end 42. In consequence, body waste such as loose waste material should be prevented from leaking out from the diaper even if a clearance is formed between the rear waist region 12 of the diaper 10 and the wearer's body. Body waste being prevented by the fold 46 from further moving toward the inner sheet's rear end 42 may move to the folded trough 47 but body waste is reliably trapped by the pocket defined by this crest and trough. In this way, body waste can be further reliably prevented from leaking out from the diaper 10.

According to this embodiment, the paired inner bond zones 81 have substantially the same length dimension and consequently the fold 46 is almost rectilinearly formed along a line extending in the transverse direction X between the opposite inner ends 81a. In other words, the fold 46 is formed substantially inparallel to the imaginary transverse center line Q-Q. As a result, the fold 46 comes in substantially even contact in the transverse direction X with the wearer's body and thereby can prevent body waste such as loose waste material from flowing in the longitudinal direction Y. In addition, the fold 46 is formed substantially in parallel to the imaginary transverse center line Q-Q, i.e., in the direction which is orthogonal to the ridges 44 and the grooves 45. The transverse stiffness of the inner sheet 40 is highest in the direction orthogonal to the ridges 44 and the grooves 45 and such a high stiffness ensures that the fold 46 maintains its shape.

The rear waist elastics 62 are attached under tension and in a contractible manner between the inner and outer sheets 40, 50 so that the inner sheet 40 also may contract in the transverse direction X under contraction of the rear waist elastics 62. The inner sheet 40 is formed with the ridges 44 and the grooves 45 and the respective ridges 44 come close to one another substantially in contact with one another so as to reduce the length dimension of the respective grooves 45 in the transverse direction X under contraction of the inner sheet 40 in the transverse direction. The ridges 44 come in contact one with another until the grooves are practically closed and eventually the clearance between the inner sheet 40 and the wearer's body substantially disappears. In this way, leak of body waste can be further reliably restricted.

The inner sheet 40 may be formed with the grooves 45 and the ridges 44, for example, by continuously ejecting gas jets from nozzles arranged above a fibrous web to this fibrous web so that the areas injected with gas may be formed with the grooves 45 and the areas not injected with gas may be formed with ridges 44. In such inner sheet 40, component fibers of the fiber web may be reoriented to make the density of the grooves 45 lower than the density of the ridges 44. By lowering the density of the grooves 45, it is possible to facilitate loose waste material to pass through the grooves 45 into the liquid-absorbent structure 20. A pressure of the gas jets may be intermittently increased to form the grooves 45 with apertures arranged intermittently. Such apertures may be formed to facilitate loose waste material or the like having a relatively high viscosity to pass therethrough.

While the inner sheet 40 is formed with the ridges 44 and the grooves 45 by means of air injection in this embodiment, the formation method of the ridges and grooves is not limited to this method so far as the fold 46 can be formed and this fold 46 has a sufficient stiffness tomaintain its shape. Specifically, it is also possible to form the inner sheet 40 with the ridges and grooves by water jet processing, steam jet processing, press working and gear working of the inner sheet 40. Various parameters such as the thickness dimension and the pitch of the ridges 44 as well as the thickness dimension of the grooves 45 are not limited to those in this embodiment and may be appropriately selected.

While the inner sheet 40 is formed on its body-facing side with the ridges 44 and the grooves 45 according to this invention, it is possible to form them on the garment-facing side thereof. However, considering some factors such as an area over which the inner sheet comes in contact with the wearer's body and perviousness for bodily fluids, the ridges 44 and the grooves 45 should be preferably formed on the body-facing side. While a so-called open-type diaper as the absorbent article has been described above, this invention is not limited to this and is applicable also, for example, to a so-called pull-on pants-type diaper.

### {Reference Signs List}

- 10: diaper (absorbent article)
- 11: front waist region
- 12: rear waist region
- 13: crotch region
- 14: chassis
- 20: liquid-absorbent structure
- 21: liquid-absorbent structure's front end
- 22: liquid-absorbent structure's rear end
- 30: containment cuffs
- 31: cuff's front end
- 32: cuff's rear end
- 33: cuff's inner side edge
- 34: cuff's outer side edge
- 36: cuff elastics
- 37: opposite side bond zones
- 38: front bond zone
- 39: rear bond zone
- 40: inner sheet
- 44: ridges
- 45: grooves
- 50: outer sheet
- 62: rear waist elastics
- 63: leg elastics
- 81: inner bond zones

## Claims

1. An absorbent article (10) having a longitudinal direction (Y) and a transverse direction (X), and comprising:
a chassis (14) comprising a body-facing side, a garment-facing side, a front waist region (11), a rear waist region (12) and a crotch region (13) extending between the front and rear waist regions (11), (12), a liquid-absorbent structure (20) lying at least in the crotch region (13), rear waist elastics (62) extending in the transverse direction along either the rear end or the front and rear ends of the chassis (14) and leg elastics (63) extending in the longitudinal direction along opposite side edges of the chassis (14) extending in the longitudinal direction; and
a pair of containment cuffs (30) lying on the body-facing side of the chassis (14) and extending in the longitudinal direction along the chassis' opposite side edges wherein the containment cuffs (30) include cuffs' front and rear ends (31), (32), cuffs' front and rear ends (31), (32) bonded to the chassis (14) by means of front and rear ends' bond zones (38), (39), cuffs' outer side edges (34) bonded to the chassis (14) by means of opposite side bond zones (37) and cuffs' inner side edges (33) elasticized so as to be contractible in the longitudinal direction and adapted to be spaced from the chassis (14), **characterized in that**:
the chassis (14) further comprises an inner sheet (40) lying on the body-facing side and an outer sheet (50) lying on the garment-facing side wherein the inner sheet (40) is contoured by front and rear ends (41), (42) extending in the transverse direction and opposite side edges (43) extending in the longitudinal direction and has a plurality of ridges (44) extending in the longitudinal direction and grooves (45) each formed between each pair of the ridges (44) being adjacent in the transverse direction;
the containment cuffs (30) are located on the body-facing side of the inner sheet (40); and the liquid-absorbent structure (20) comprises front and rear ends (21), (22) extending in the transverse direction, a spacing zone (80) is defined between the inner sheet's rear end (42) and the liquid-absorbent structure's rear end (22) in the longitudinal direction, and the cuffs' inner side edges (33) are bonded to the inner sheet (40) by means of a pair of inner bond zones (81) formed in said spacing zone (80), wherein outer ends (81b) of said inner bond zones (81) are defined at positions corresponding to the rear end (42) of the inner sheet (40) and inner ends (81a) of said inner bond zones (81) are positioned in said spacing zone (80) between said rear waist elastics (62) and said liquid-absorbent structure's rear end (22).

2. The absorbent article (10) defined by claim 1, wherein said inner and outer sheets (40), (50) respectively include front and rear ends (41), (42), (51), (52) and side edges (43), (53) wherein a length dimension of said inner sheet (40) in said transverse direction is smaller than that of said outer sheet (50) so that said side edges (53) of said outer sheet extend outward beyond said side edges (43) of said inner sheet, said cuffs' outer side edges (34) extend outward in said transverse direction beyond said associated side edges (43) of the inner sheet and bonded to said associated side edges (53) of the outer sheet by means of associated side bond zones (37), and said leg elastics (63) are secured between said cuffs' outer side edges (34) and said side edges (53) of said outer sheet (50) also by means of said associated side bond zones (37).

3. The absorbent article (10) defined by claim 1 or 2, wherein the containment cuffs (30) are elasticized in the longitudinal direction by cuff elastics (36) attached to the cuffs' inner side edges (33) and the cuff elastics (36) are attached to the cuffs' inner side edges (33) inside the inner bond zones (81) as viewed in the longitudinal direction.

4. The absorbent article (10) defined by any one of claims 1 through 3, wherein the paired inner bond zones (81) have the same length dimension as measured from the inner sheet's rear end (42) in the longitudinal direction.

5. The absorbent article (10) defined by any one of claims 1 through 4, wherein the inner sheet (40) has a transverse stiffness against bending in the direction orthogonal to the ridges (44) and the grooves (45) higher than a longitudinal stiffness against bending along the ridges (44) and the grooves (45) wherein the transverse stiffness is more than or equal to 50mm and less than 90mm.

6. The absorbent article (10) defined by any one of claims 1 through 5, wherein the rear waist elastics (62) are attached between the inner sheet (40) and the outer sheet (50).

## Patentansprüche

1. Saugfähiger Artikel (10), der eine Längsrichtung (Y) und eine Querrichtung (X) hat, und Folgendes umfasst:
einen Rahmen (14), der eine in Richtung des Körpers zeigende Seite, eine in Richtung des Kleidungsstücks zeigende Seite, einen vorderen Taillenbereich (11), einen hinteren Taillenbereich (12) und einen Schrittbereich (13) hat, die sich zwischen der den vorderen und hinteren Taillenbereichen (11), (12) erstrecken, eine flüssigkeitsabsorbierende Struktur (20), die wenigstens im Schrittbereich (13) liegt, hintere Taillengummibänder (62), die sich in Querrichtung entweder am hinteren Ende oder am vorderen und hinteren Ende des Rahmens (14) erstrecken und Beingummibänder (63) hat, die sich in Längsrichtung auf den gegenüberliegenden Seitenrändern des Rahmens (14) erstrecken, und sich in Längsrichtung erstrecken,
und
ein paar Haltemanschetten (30), die auf den dem Körper zugewandten Seiten des Rahmens (14) liegen und sich in Längsrichtung an den gegenüberliegenden Seitenrändern des Rahmens erstrecken, wobei die Haltemanschetten (30) das vordere und hintere Ende (31), (32) der Manschetten beinhalten, das vordere und hintere Ende (31), (32) der Manschetten, die mit dem Rahmen (14) durch die Verbindungszonen der vorderen und hinteren Enden (38), (39) und den Außenrändern der Manschetten (34) mit dem Rahmen (14) verbunden sind, durch Mittel der gegenüberliegenden Verbindungszonen (37) und die inneren Seitenränder (33) der Manschette mit Gummizug ausgestattet sind, sodass sie in Längsrichtung zusammenziehbar und so adaptiert sind, dass sie vom Rahmen (14) auseinanderliegen und so gekennzeichnet sind, dass
der Rahmen (14) ferner eine innere Schicht (40) umfasst, die auf der dem Körper zugewandten Seite liegt, und eine äußere Schicht (50), die auf der dem Kleidungsstück zugewandten Seite liegt, wobei die innere Schicht (40) am vorderen und hinteren Ende (41), (42) konturiert ist, und sich in Querrichtung ersteckt, und sich die gegenüberliegenden Seitenränder (43) in Längsrichtung erstrecken und eine Vielzahl von Erhöhungen (44) hat, die sich in Längsrichtung erstrecken, und Vertiefungen (45), von denen jede zwischen jedem Paar Erhöhungen (44) angrenzend in Querrichtung ist,
die Haltemanschetten (30) auf der dem Körper zugewandten Seite der inneren Schicht (40) angebracht sind, und die flüssigkeitsabsorbierende Struktur (20) vordere und hintere Enden (21), (22) umfasst, die sich in Querrichtung erstrecken, eine Abstandszone (80), die zwischen dem hinteren Ende (42) der inneren Schicht und dem hinteren Ende (22) der flüssigkeitsabsorbierenden Struktur in Längsrichtung, und den inneren Seitenrändern (33) der Manschetten mit der inneren Schicht (40) verbunden sind, mithilfe eines Paares von inneren Verbindungszonen (81), die in den Abstandszonen (80) geformt sind, wobei die äußeren Enden (81 b) der inneren Verbindungszonen (81) an Positionen definiert sind, die mit dem hinteren Ende (42) der inneren Schicht (40) korrespondieren, und die inneren Enden (81 a) der inneren Verbindungszonen (81) in der Abstandszone (80) positioniert sind, und zwar zwischen dem hinteren Taillengummi (62) und dem hinteren Ende der flüssigkeitsabsorbierenden Struktur (22).

2. Saugfähiger Artikel (10) nach Anspruch 1, wobei die inneren und äußeren Schichten (40), (50) jeweils vordere und hintere Enden (41), (42), (51), (52) und Seitenränder (43), (53) beinhalten, und wobei ein Längenmaß der inneren Schicht (40) in Querrichtung kleiner ist als die der äußeren Schicht (50), sodass sich die Seitenränder (53) der äußeren Schicht nach außen über beide Seitenränder (43) der inneren Schicht erstrecken, wobei die äußeren Seitenränder (34) der Manschetten nach außen in Querrichtung über die begleitenden Seitenränder (43) der inneren Schicht erstrecken und mit den begleitenden Seitenränder (53) der äußeren Schicht mithilfe der begleitenden Seitenverbindungszonen (37) verbunden sind, und der Beingummi (63) zwischen den außeren Seitenrändern (34) der Manschetten gesichert sind, und die Seitenränder (53) der äußeren Schicht (50) auch mithilfe der begleitenden Seitenverbindungszonen (37) gehalten werden.

3. Saugfähiger Artikel (10) nach Anspruch 1 oder 2, wobei die Haltemanschetten (30) einen Gummizug in Längsrichtung des Manschettengummis (36) haben, die an den inneren Seitenrändern (33) der Manschette angebracht sind und der Manschettengummi (36) an den inneren Seitenrändern (33) der Manschetten angebracht sind, und zwar innerhalb der inneren Verbindungszonen (81), wenn in Längsrichtung betrachtet.

4. Saugfähiger Artikel (10) nach einem der Ansprüche 1 bis 3, wobei die gepaarten inneren Verbindungszonen (81) dieselben Längenmaße haben, wie vom hinteren Ende der inneren Schicht (42) in Längsrichtung gemessen.

5. Saugfähiger Artikel (10) nach einem der Ansprüche 1 bis 4, wobei die innere Schicht (40) eine Quersteifigkeit gegen Biegen in rechtwinkliger Richtung zu den Erhöhungen (44) hat, und die Vertiefungen (45) höher as eine Längssteifigkeit gegen Biegen an den Erhöhungen (44) und den Vertiefungen (45) entlang, wobei die Quersteifigkeit höher oder gleich 50 mm und weniger als 90 mm ist.

6. Saugfähiger Artikel (10) nach einem der Ansprüche 1 bis 5, wobei die hinteren Taillengummis (62) zwischen der inneren Schicht (40) und der äußeren Schicht (50) angebracht sind.

## Revendications

1. Article absorbant (10) ayant une direction allant dans le sens longitudinal (Y) et une direction allant dans le sens transversal (X), et comportant :
une armature (14) comportant un côté orienté vers le corps, un côté orienté vers le vêtement, une région avant au niveau de la taille (11), une région arrière au niveau de la taille (12) et une région au niveau de l'entrejambe (13) s'étendant entre les régions avant et arrière au niveau de la taille (11), (12), une structure absorbant les liquides (20) reposant au moins dans la région au niveau de l'entrejambe (13), des élastiques au niveau de la taille côté arrière (62) s'étendant dans la direction allant dans le sens transversal le long soit de l'extrémité arrière soit des extrémités avant et arrière de l'armature (14) et des élastiques au niveau des jambes (63) s'étendant dans la direction allant dans le sens longitudinal le long de bords latéraux opposés de l'armature (14) s'étendant dans la direction allant dans le sens longitudinal ; et
une paire de revers de retenue (30) reposant sur le côté orienté vers le corps de l'armature (14) et s'étendant dans la direction allant dans le sens longitudinal le long des bords latéraux opposés de l'armature dans lequel les revers de retenue (30) comprennent des extrémités avant et arrière (31), (32) des revers, des extrémités avant et arrière (31), (32) des revers reliées sur l'armature (14) au moyen de zones de liaison (38), (39) des extrémités avant et arrière, des bords latéraux extérieurs (34) des revers reliés sur l'armature (14) au moyen de zones de liaison latérales opposées (37) et des bords latéraux intérieurs (33) des revers munis d'élastiques de manière à pouvoir être contractés dans la direction allant dans le sens longitudinal et adaptés pour être espacés par rapport à l'armature (14), **caractérisé en ce que** :
l'armature (14) comporte par ailleurs une feuille intérieure (40) reposant sur le côté orienté vers le corps et une feuille extérieure (50) reposant sur le côté orienté vers le vêtement dans lequel la feuille intérieure (40) est profilée par des extrémités avant et arrière (41), (42) s'étendant dans la direction allant dans le sens transversal et des bords latéraux opposés (43) s'étendant dans la direction allant dans le sens longitudinal et a une pluralité de nervures (44) s'étendant dans la direction allant dans le sens longitudinal et des rainures (45) dont chacune est formée entre chaque paire des nervures (44) adjacentes dans la direction allant dans le sens transversal ;
les revers de retenue (30) sont situés sur le côté orienté vers le corps de la feuille intérieure (40) ; et la structure absorbant les liquides (20) comporte des extrémités avant et arrière (21), (22) s'étendant dans la direction allant dans le sens transversal, une zone d'espacement (80) est définie entre l'extrémité arrière (42) de la feuille intérieure et l'extrémité arrière (22) de la structure absorbant les liquides dans la direction allant dans le sens longitudinal, et les bords latéraux intérieurs (33) des revers sont reliés à la feuille intérieure (40) au moyen d'une paire de zones de liaison intérieures (81) formées dans ladite zone d'espacement (80), dans lequel des extrémités extérieures (81b) desdites zones de liaison intérieures (81) sont définies au niveau de positions correspondant à l'extrémité arrière (42) de la feuille intérieure (40) et les extrémités intérieures (81a) desdites zones de liaison intérieures (81) sont positionnées dans ladite zone d'espacement (80) entre lesdites élastiques au niveau de la taille côté arrière (62) et ladite extrémité arrière (22) de la structure absorbant les liquides.

2. Article absorbant (10) selon la revendication 1, dans lequel lesdites feuilles intérieure et extérieure (40), (50) comprennent respectivement des extrémités avant et arrière (41), (42), (51), (52) et des bords latéraux (43), (53) dans lequel une dimension de longueur de ladite feuille intérieure (40) dans ladite direction allant dans le sens transversal est inférieure par rapport à celle de ladite feuille extérieure (50) de telle sorte que lesdits bords latéraux (53) de ladite feuille extérieure s'étendent vers l'extérieur au-delà desdits bords latéraux (43) de ladite feuille intérieure, lesdits bords latéraux extérieurs (34) des revers s'étendent vers l'extérieur dans ladite direction allant dans le sens transversal au-delà desdits bords latéraux associés (43) de ladite feuille intérieure et reliés auxdits bords latéraux associés (53) de la feuille extérieure au moyen de zones de liaison latérales associées (37), et lesdits élastiques au niveau des jambes (63) sont assujettis entre lesdits bords latéraux extérieurs (34) des revers et lesdits bords latéraux (53) de ladite feuille extérieure (50) également au moyen desdites zones de liaison latérales associées (37).

3. Article absorbant (10) selon la revendication 1 ou la revendication 2, dans lequel les revers de retenue (30) sont munis d'élastiques dans la direction allant dans le sens longitudinal par des élastiques (36) de revers attachés sur les bords latéraux intérieurs (33) des revers et les élastiques (36) de revers sont attachés sur les bords latéraux intérieurs (33) des revers à l'intérieur des zones de liaison intérieures (81) tel que l'on peut voir dans la direction allant dans le sens longitudinal.

4. Article absorbant (10) selon l'une quelconque des revendications 1 à 3, dans lequel les zones de liaison intérieures appariées (81) ont la même dimension de longueur telle qu'elle est mesurée depuis l'extrémité arrière (42) de la feuille intérieure dans la direction allant dans le sens longitudinal.

5. Article absorbant (10) selon l'une quelconque des revendications 1 à 4, dans lequel la feuille intérieure (40) a une rigidité transversale contre toute flexion dans la direction perpendiculaire par rapport aux nervures (44) et aux rainures (45) supérieure par rapport à une rigidité longitudinale contre toute flexion le long des nervures (44) et des rainures (45) dans lequel la rigidité transversale est supérieure ou égale à 50 mm et inférieure à 90 mm.

6. Article absorbant (10) selon l'une quelconque des revendications 1 à 5, dans lequel les élastiques au niveau de la taille côté arrière (62) sont attachés entre la feuille intérieure (40) et la feuille extérieure (50).
